# EUROPEAN PATENT APPLICATION

(11) **EP 3 841 976 A1**
(43) Date of publication of application: **30.06.2021**
(21) Application number: 20215940.6
(22) Date of filing: 21.12.2020
(51) Int. Cl.: A61B 5/257, A61B 5/27, A61B 5/271

(54) **PRINTED ELECTROCARDIOGRAM LEADS FOR MEDICAL APPLICATIONS**

(30) Priority: 23.12.2019 US 201962953131 P; 18.12.2020 US 202017126476
(71) Applicant: Nikomed USA, Inc., Hatboro, Pennsylvania 19040 (US)
(72) Inventor: Epstein, Stephen T., Newtown, Pennsylvania 18940 (US)
(74) Representative: MacLachlan & Donaldson

(57) **Abstract**

An electrocardiogram lead package that contains low-cost printed constructs and the associated method of manufacture. The electrocardiogram lead package contains a first and second printed construct. The constructs include electrodes, lead hubs, and printed wire leads on a flexible substrate. The electrodes contain printed contact heads. The lead hubs contain printed contact pads. The printed wire leads contains printed conductive pathways. On the flexible substrate, the electrodes of the two constructs are linearly aligned and interposed. When separated from the electrocardiogram lead package, the first and second printed constructs provide all the electrodes and leads needed to perform an electrocardiogram. After one use, the printed constructs are discarded.

## Description

### RELATED APPLICATIONS

The present application claims the benefit of U.S. Provisional Application No. 62/953,131, filed December 23, 2019.

### BACKGROUND OF THE INVENTION

### 1. Field Of The Invention

In general, the present invention relates to the structure of electrocardiogram lead elements that interconnect a patient to medical analysis equipment. The present invention also relates to methods of forming electrocardiogram lead elements using printing manufacturing techniques.

### 2. Prior Art Description

Many medical testing and monitoring systems require that various probes and sensors be attached to the body of a patient. For example, to perform an electrocardiogram, multiple sensors are attached to the torso and limbs. A common type of electrocardiogram (ECG) is the 10-lead ECG. The 10-lead ECG gives a tracing of the heart from multiple directions using a series of ten electrodes. Each of the ten electrodes picks up electrical activity from a different position on the heart muscle. This allows an experienced interpreter to see the heart from many different angles. The actual electrocardiogram is a graphic record of the direction and magnitude of the electrical activity generated by the depolarization and repolarization of the atria and ventricles of the heart. This electrical activity is readily detected by the ten electrodes attached to the skin.

An ECG utilizes electrodes of opposite polarity (one positive and one negative) or one positive surface electrode and a reference point. Two electrodes of opposite polarity are called a bipolar leads. A lead composed of a single positive electrode and a reference point is a unipolar lead. A ten-lead ECG consists of various bipolar limb leads, unipolar limb leads, and unipolar chest leads, which are also called precordial or V leads.

In each case, the wire leads attach to disposable electrodes that adhere to the limbs and torso of a patient. The wire leads are difficult to manage as ten electrodes are applied to the body. Furthermore, ten wire leads must be properly attached to the correct electrodes. The wire leads often become entangled, therein causing an error during the application.

Wire leads for medical equipment, such as electrocardiograms, are complicated assemblies that are rarely replaced. Rather, many hospitals, clinics and physicians' offices only use disposable electrodes and repeatedly reuse the wire leads that connect the electrodes to the medical equipment. This, of course, presents problems with patient-to-patient contamination. Wire leads come into contact with a patient's skin and clothing. As such, wire leads can be contaminated with bacteria, viruses, blood and/or other bodily fluids after one use. As a consequence, healthcare providers are required to balance the risks and costs associated with replacing or reusing wire leads. Healthcare providers must either absorb the large expense of replacing or sterilizing wire leads after each use, or they must assume the dangers and complications of potentially cross-contaminating patients by reusing the wire leads.

Another disadvantage of traditional wire leads is that the connectors at the ends of the wire leads wear out over time. As the connectors wear, they often become loose and create weak electrical connections with the probe or sensor to which it is attached. Loose connections create noise and disruptions in the electrical signals being sensed. As such, one or more loose connections can invalidate an electrocardiogram test.

In the prior art, attempts have been made to replace expensive wire leads with less expensive elements, such as printed flexible substrates. Such prior art is exemplified by U.S. Patent No. 6,006,125 to Kelly. One problem associated with such printed substrates, is that they are printed in one size in the hope that one size will fit all people. This is clearly not accurate. An infant is obviously very different in size than a full-grown adult. Wire leads can be manipulated to accommodate people of different sizes. However, printed leads are fixed on a substrate. As such, the premanufactured printed leads must be produced in a wide variety of sizes and styles to accommodate people of different ages, sizes, shapes and genders. This requires preprinted substrates of many different sizes and lengths to be held in the inventory of a hospital or clinic. The consequence is that large sums of money must be spent on inventory. This negates the cost savings of not using traditional lead wires.

In an attempt to produce a low cost wiring harness that can be used on patients of most any size, wire leads have been printed on substrates where each wire lead is separate and distinct. This enables each of the wire leads to be independently positioned on a patient's body. Such prior art is exemplified by U.S. Patent Application Publication No. 2011/0054286 to Crosby. A problem with such printed leads is that the leads must be connected to electrodes at one end and the medical equipment cable at the opposite end. As such, if each lead has two connections and there are ten leads, a 10-lead ECG system has twenty separate electrical connections that must be maintained during a procedure. If one or more connections become loose, the electrocardiogram test is compromised. Furthermore, it takes a long period to diagnose the connections and find the bad connection before the procedure can be restarted.

In the prior art, there are wiring harnesses, where the wire leads are premanufactured to be connected to the electrodes. As such, there is no chance of a poor connection between the wire leads and the electrodes. Such prior art is exemplified by U.S. patent No. 4,353,372 to Ayer. The problem with such prior art systems is that the integration of the wire leads within the electrodes creates a complicated structure that cannot be simply printed on a printing machine. The complicated structure increases the complexities and costs of manufacture. The more expensive a system is, the less likely such a system will be purchased as disposable.

A need therefore exists for a new construct and method of manufacture that enables a wiring harness to be made using only low-cost printing manufacturing techniques, wherein the wiring harness contains both electrodes and the wire leads that connect the wire leads to a medical machine. A need also exists for a wiring harness with electrodes and wire leads that is highly reliable, yet inexpensive enough to be replaced after every use. These needs are met by the present invention as described and claimed below.

### SUMMARY OF THE INVENTION

The present invention is an electrocardiogram lead package that contains low-cost printed constructs and the associated method of manufacturing the printed constructs. The electrocardiogram lead package contains a first and second printed construct. The first printed lead construct includes a first plurality of electrodes, a first lead hub, and a first plurality of printed wire leads that electrically interconnect the first plurality of electrodes to the first lead hub. The first plurality of electrodes contains a first set of printed contact heads. The first lead hub contains a first set of printed contact pads. The first plurality of printed wire leads contains a first set of printed conductive pathways.

The second printed lead construct has a second plurality of electrodes, a second lead hub, and a second plurality of printed wire leads that electrically interconnect the second plurality of electrodes to the second lead hub. The second plurality of electrodes contains a second set of printed contact heads. The second lead hub contains a second set of printed contact pads. The second plurality of printed wire leads contains a second set of printed conductive pathways. The first and second sets of printed contact heads, the first and second sets of printed contact pads, and the first and second sets of printed conductive pathways are all formed from conductive ink that is printed onto a common segment of flexible substrate.

On the flexible substrate, the first plurality of electrodes are linearly aligned, and interposed with, the second plurality of electrodes. When separated from the electrocardiogram lead package, the first and second printed constructs provide all the electrodes and leads needed to perform an electrocardiogram. After one use, the printed constructs are thrown away.

### BRIEF DESCRIPTION OF THE DRAWINGS

For a better understanding of the present invention, reference is made to the following description of an exemplary embodiment thereof, considered in conjunction with the accompanying drawings, in which:
FIG. 1 shows the present invention being used to connect a patient to an electrocardiogram machine;
FIG. 2 is a top view of printed constructs on a common substrate;
FIG. 3 is a cross-sectional view of a section of a printed construct;
FIG. 4 shows and enlarged section of an electrode with an alternate construction of a printed wire lead; and
FIG. 5 shows printed constructs separated and ready for use; and
FIG. 6 is a block diagram outlining the method of manufacture.

### DETAILED DESCRIPTION OF THE DRAWINGS

Although the present invention system and method can be embodied in many ways, only one exemplary embodiment is shown. This embodiment is selected in order to set forth one of the best modes contemplated for the invention. The illustrated embodiment, however, is merely exemplary and should not be considered a limitation when interpreting the scope of the appended claims.

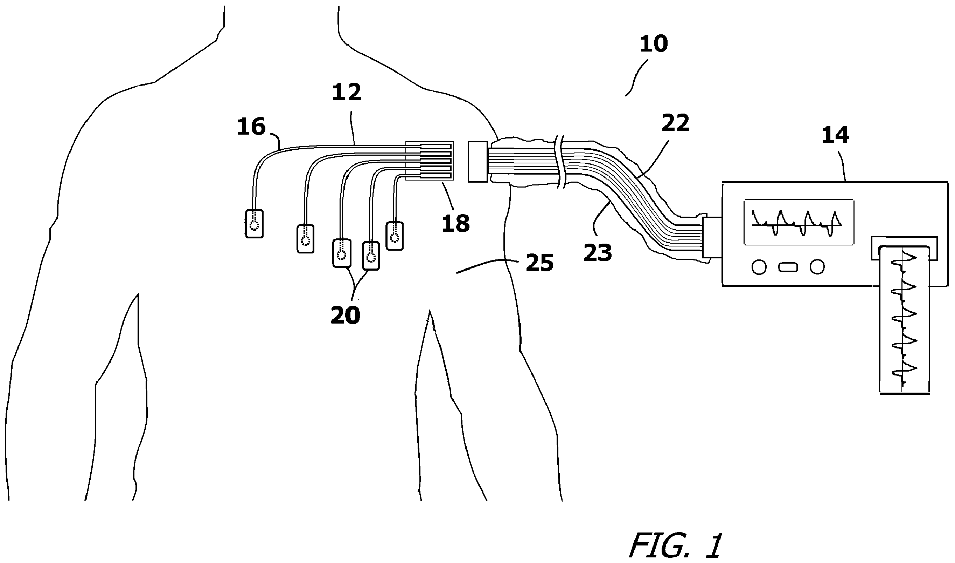

Referring to Fig. 1, an application 10 is shown where printed constructs 12 are used to interconnect an electrocardiogram machine 14 to a patient. During an electrocardiogram, two such printed constructs 12 would be used. One is shown for simplicity. The printed constructs 12 each contain five electrodes 20 that are integrated with five printed wire leads 16. As such, two printed constructs 12 would contain ten electrodes 20 and ten printed wire leads 16, wherein the two printed constructs 12 can be utilized to perform a multi-lead electrocardiogram.

Each electrode 20 present on a printed construct 12 has its own independent printed wire leads 16. The printed wire leads 16 connect the electrodes 20 to a common lead connection hub 18. The lead connection hub 18 enables each of the printed constructs 12 to interconnect to a multi-lead cable 22. The multi-lead cable 22 interconnects the printed constructs 12 to the electrocardiogram machine 14. As will be explained, it is only the printed constructs 12 that contact the body 25 of the patient. The multi-lead cable 22 can be kept away from the body 25 of the patient or can be sheathed in a disposable plastic sleeve 23. Accordingly, the printed constructs 12 are for single use applications and the multi-lead cable 22 can be used repeatedly.

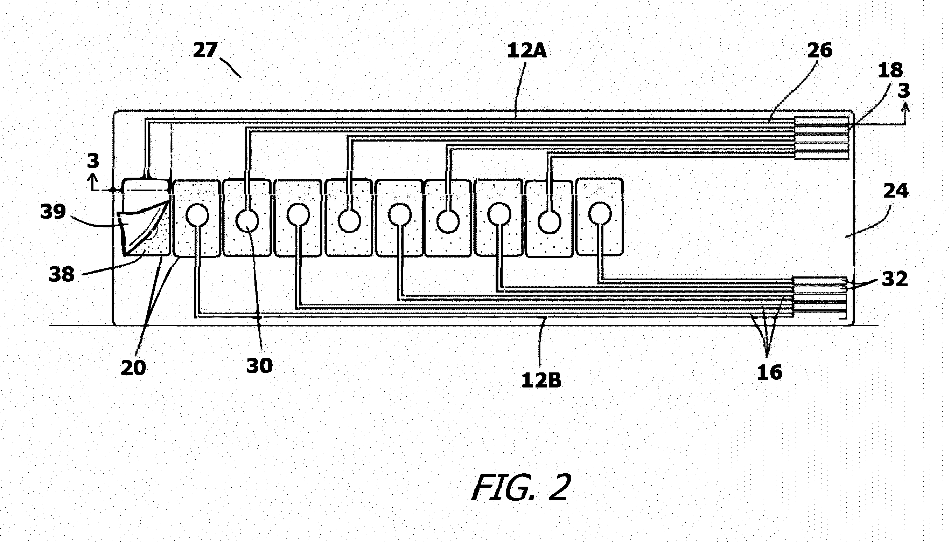

Referring to Fig. 2 in conjunction with Fig. 1, it can be seen that two printed constructs 12 are printed upon a single segment of flexible substrate 24 to form a complete ECG lead package 27. The two printed constructs 12 in the ECG lead package 27 include a first printed construct 12A and a second printed construct 12B. As a result, each ECG lead package 27 contains ten electrodes 20, ten printed wire leads 16, and two lead connection hubs 18 that are all formed on a common segment of flexible substrate 24. In this manner, the ECG lead package 27 is manufactured and packaged as a unit for single use by a healthcare professional. A healthcare professional removes the ECG lead package 27 from sterile packaging (not shown) and separates the two printed constructs 12 from the remaining flexible substrate 24. The printed constructs 12 are then applied to the body 25 of the patient for use in performing an electrocardiogram. As will be explained, the positioning of the printed constructs 12 are variable and are governed by the size and shape of the patient's body 25. After use, the printed constructs 12 are removed from the body 25 and discarded.

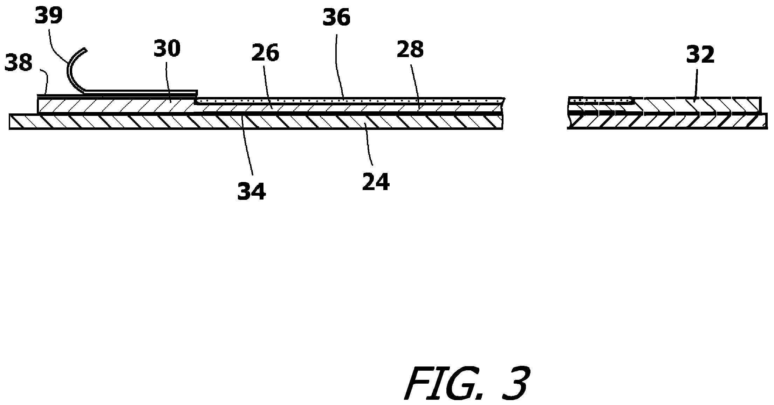

Referring to Fig. 3, in conjunction with Fig. 2 and Fig. 1, it will be understood that to create the printed constructs 12, a single flexible substrate 24 is provided. The flexible substrate 24 is dielectric and can be either polymer-based or paper-based. The preferred flexible substrate 24 is a film of polyethylene terephthalate (PET). However, films of flashspun non-woven high-density polyethylene fiber, such as Tyvek®, can also be used. These films are dielectric, highly flexible, and tear-resistant in tension.

In a first printing operation, conductive ink 26 is printed upon the flexible substrate 24. The conductive ink 26 is printed in a precise pattern to form the conductive features of each printed construct 12. The conductive features include contact heads 30 within the electrodes 20, contact pads 32 within the lead connection hubs 18, and conductive pathways 28 within the printed wire leads 16.

The contact pads 32 for each printed construct 12 are all arranged in parallel in the same areas, therein forming the common lead connector hubs 18. The contact heads 30 are linearly aligned, but are printed at different distances from the contact pads 32. The contact heads 30 of the first printed construct 12A and the contact heads 30 of the second printed construct 12B are interposed and linearly aligned. In this manner, the density of useful elements on the flexible substrate 24 are maximized. This reduces the area of the flexible substrate 24 needed to support the ECG package 27 and minimizes waste.

Since the printed contact heads 30 are all different distances from the printed contact pads 32, it will be understood that the length of each of the conductive pathways 28 between the contact heads 30 and the contact pads 32 is different. The lengths of the various conductive pathways 28 are designed to be long enough to reach the proper attachment point on the patient's body 25 during an electrocardiogram, regardless of the size of the patient. Each conductive pathway 28 is electrically isolated from the other conductive pathways 28 printed on the same flexible substrate 24. Likewise, the contact heads 30 are electrically isolated from one another, as are the contact pads 32.

The contact heads 30, the contact pads 32 and the conductive pathways 28 are all printed in the same printing operation. As such, the contact heads 30, conductive pathways 28, and contact pads 32 are all electrically interconnected without the need of any connections between features. The contact heads 30, conductive pathways 28 and contact pads 32 are all a common deposit of the conductive ink 26 that is printed upon the material of the flexible substrate 24. The conductive ink 26 is preferably applied using an industrial grade electronic printer or a 3-D printer. However, alternate printing methods, such as silkscreening, can also be used. Many types of conductive inks can be used in the printing. However, to limit distortion and cracking of the conductive ink, silver-based inks are preferred. The preferred silver-based ink is an Ag/AgCI ink. An Ag/AgCI ink transfers an electrical charge across its boundaries by a reversible redox reaction. Accordingly, an Ag/AgCI ink will not polarize and will not filter or otherwise alter the electrical signal during an electrocardiogram.

Depending upon the composition of the flexible substrate 24 and the composition of the conductive ink 26, the flexible substrate 24 may be coated with an aqueous primer 34 that increases the adhesion between the conductive ink 26 and the flexible substrate 24. There are many primers commercially available that are used to print ink onto PET or high-density polyethylene fiber. Many of these primers work with silver-based conductive inks and can be incorporated into the present invention. The primer 34 prevents the conductive ink 26 from peeling away from the flexible substrate 24 if the flexible substrate 24 is severely deformed during processing and/or use. The primer 34 is also beneficial in the adhesion of any insulation layer over the flexible substrate 24 and conductive ink 26.

A dielectric insulation layer 36 is applied over some areas of the conductive ink 26 and the flexible substrate 24. The dielectric insulation layer 36 is not applied over the contact heads 30 or the contact pads 32. The dielectric insulation layer 36 can be applied in one of two ways. The dielectric insulation layer 36 can simply be a layer of dielectric ink that is printed over the conductive ink 26 and the exposed flexible substrate 24. The dielectric ink encapsulates the conductive ink 26 so that the conductive ink 26 is interposed between the flexible substrate 24 and the dielectric ink. Alternatively, the dielectric insulation layer 36 can be a curable dielectric polymer that is sprayed or otherwise mechanically applied over the conductive ink 26 and the exposed flexible substrate 24. In either manufacturing scenario, the conductive ink 26 is interposed between the flexible substrate 24 and the first dielectric insulation layer 36. Accordingly, the insulated areas of the conductive ink 26 cannot short against the skin or any metallic object that it may inadvertently contact.

The dielectric insulation layer 36 is not applied over the contact heads 30. A thin layer of tacky adhesive 38 is applied around each contact head 30 in the areas that will form the electrode 20. If the tacky adhesive 38 is conductive, it can be applied over the contact heads 30. If the tacky adhesive 38 is not conductive, it is applied around the contact heads 30. After the tacky adhesive 38 is applied, the tacky adhesive 38 is covered by a peel away protective cover 39. This prevents the tacky adhesive 38 from becoming contaminated with dust, dirt and oil, prior to use.

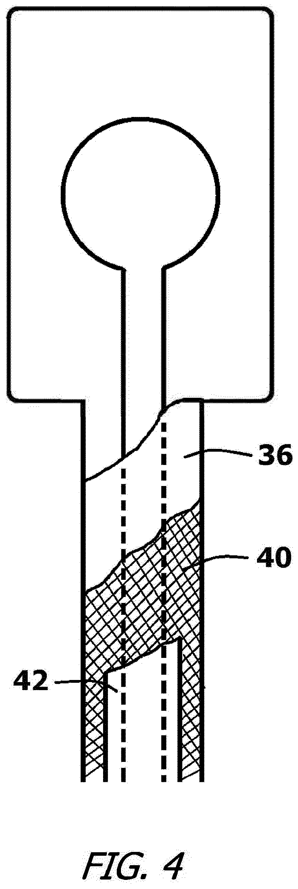

Referring to Fig. 4, it can be seen that an optional shielding layer 40 can be printed atop the first dielectric insulation layer 36. The shielding layer 40 is made from conductive ink and can be applied either as a solid layer or as a mesh layer. The shielding layer 40 helps prevent the conductive pathways from receiving noise signals from external electromagnetic sources. The shielding layer 40 itself can be covered with a printed second dielectric insulation layer 42 to prevent the shielding layer 40 from receiving any signals from inadvertent contact with metal objects or static discharge.

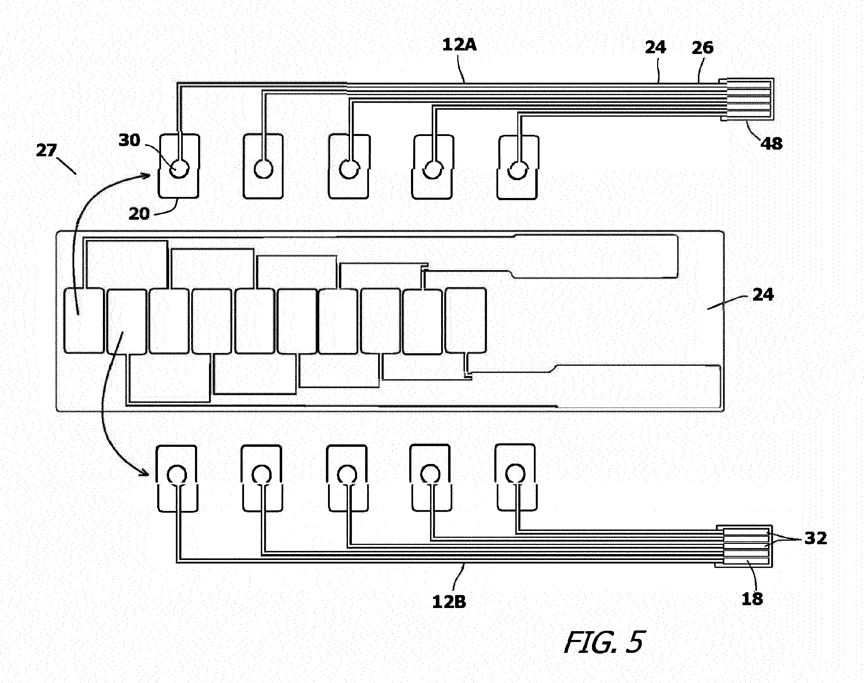

Referring to Fig. 5 in conjunction with Fig. 2 and Fig. 3, it can be seen that the flexible substrate 24 is stamped to define the peripheries of the first printed construct 12A and the second printed construct 12B within the ECG lead package 27. The stamping can leave small areas of attachment (not shown) that are easily broken by manual manipulation. In this manner, the ECG lead package 27 keeps its shape until it is unpackaged for use.

When the flexible substrate 24 is stamped, large bases 46 are left around the contact heads 30. The bases 46 are the shape of the final electrode 20. Furthermore, the layer of tacky adhesive 38 is applied across the full area of each base 46. Accordingly, each electrode 20 is made from a base 46 stamped out of the flexible substrate 24. In the center of the base 46 is a printed contact head 30. The base 46 is coated with tacky adhesive 38 and covered in a peel-away cover layer 39.

Likewise, the substrate 24 is not cut in a wide base area 48 surrounding the contact pads 32. The contact pads 32 printed on the base area 48 form the lead connection hub 18 for each of the printed constructs 12. Furthermore, tracks 50 are left around the conductive pathways 28 to support the lines of conductive ink 26. Accordingly, each of the conductive pathways 28 is a stamped track 50 of the flexible substrate 24 that is printed with conductive ink 26 and coated with a first insulation layer 36. The first insulation layer 36 may have a shielding layer 40 printed upon it and a secondary dielectric insulation layer 42 covering the shielding layer 40.

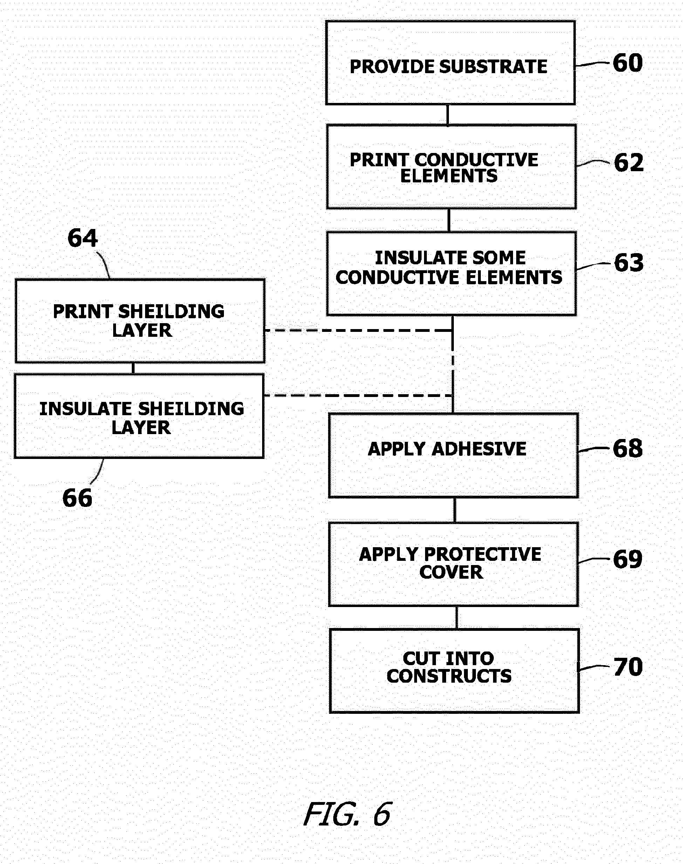

The printed constructs 12 are printed upside down. Accordingly, the last elements printed lay closest to the patient's body when applied to the body. Referring to Fig. 6 in conjunction with all previous figures, it will be understood that to manufacture the ECG lead package 27, a segment of flexible substrate 24 is provided. See Block 60. The contact heads 30, conductive pathways 28 and contact pads 32 are printed onto the flexible substrate 24 in conductive ink 26. See Block 62. The conductive ink 26 in the conductive pathways 28 are covered in a first dielectric insulation layer. See Block 63. Optionally, the first dielectric insulation layer can be printed with a shielding layer and a subsequent second dielectric insulation layer. See Block 64 and Block 66.

The areas surrounding the contact heads 30 are coated with a tacky adhesive 38. See Block 68. The tacky adhesive 38 is then covered with a protective peel-away cover layer 39. See Block 69. The printed assembly is then stamped to cut along the peripheries of the two printed constructs 12. See Block 70. The two printed constructs 12 remain attached to the remaining flexible substrate 24 by a few areas of attachment 44 left after the stamping. The result is an ECG lead package 27 that can packaged, shipped and sold.

Once needed, the ECG lead package is removed from its packaging and the printed constructs 12 are removed and detached. To use the printed constructs 12, the peel-away cover layers 39 are peeled away from the electrodes 20, therein exposing the tacky adhesive 38. The electrodes 20 are then attached to a patient's body 25 in the areas appropriate for an electrocardiogram, give the shape and size of the body 25. This brings the contact heads 30 into electrical contact with the body 25. The printed wire leads 16 extend away from the patient and terminate at the lead connector hubs 18. As shown in Fig. 1, the lead connectors 18 are attached to multi-lead cables 22 that extend to the electrocardiogram machine 14. After use, the printed constructs 12 can be removed from the patient and discarded.

It will be understood that the embodiment of the present invention that is illustrated and described is merely exemplary and that a person skilled in the art can make many variations to that embodiment. All such embodiments are intended to be included within the scope of the present invention as defined by the claims.

## Claims

1. An electrocardiogram lead package assembly, comprising:
a first printed lead construct having a first plurality of electrodes, a first lead hub, and a first plurality of printed wire leads that electrically interconnect the first plurality of electrodes to the first lead hub, wherein the first plurality of electrodes contain a first set of printed contact heads, the first lead hub contains a first set of printed contact pads and the first plurality of printed wire leads contains a first set of printed conductive pathways;
a second printed lead construct having a second plurality of electrodes, a second lead hub, and a second plurality of printed wire leads that electrically interconnect the second plurality of electrodes to the second lead hub, wherein the second plurality of electrodes contain a second set of printed contact heads, the second lead hub contains a second set of printed contact pads and the second plurality of printed wire leads contains a second set of printed conductive pathways;
wherein the first set of printed contact heads, the second set of printed contact heads, the first set of printed contact pads, the second set of printed contact pads, the first set of printed conductive pathways, and the second set of printed conductive pathways are all formed from conductive ink printed onto a common segment of flexible substrate.

2. The assembly according to claim 1, further including a dielectric insulation layer applied over the conductive ink that forms first set of printed conductive pathways and the second set of printed conductive pathways.

3. The assembly according to Claim 1, wherein the first plurality of electrodes and the second plurality of electrodes include adhesive applied to the common segment of flexible substrate.

4. The assembly according to Claim 1, wherein the first plurality of electrodes includes at least five separate electrodes.

5. The assembly according to Claim 4, wherein the second plurality of electrodes includes at least five separate electrodes.

6. The assembly according to Claim 5, wherein the first plurality of electrodes are linearly aligned with the second plurality of electrodes on the common segment of flexible substrate.

7. The assembly according to Claim 6, wherein the first plurality of electrodes are interposed with the second plurality of electrodes on the common segment of flexible substrate.

8. The construct according to Claim 1, wherein each of the first set of printed conductive pathways extends a different length.

9. The construct according to Claim 8, wherein each of the second set of printed conductive pathways extends a different length.

10. The construct according to Claim 9, wherein the first set of printed conductive pathways are different lengths than the second set of printed conductive pathways.

11. An electrocardiogram lead construct, comprising:
flexible substrate;
contact heads disposed on the flexible substrate;
contact pads disposed on the flexible substrate;
conductive pathways disposed on the flexible substrate that electrically interconnect the contact heads to the contact pads, wherein the contact heads, the contact pads and the conductive pathways are all formed from a common deposit of conductive ink printed onto the flexible substrate;
a dielectric insulation layer applied over the common deposit of conductive ink that forms the conductive pathways;
adhesive applied to the flexible substrate in areas surrounding the contact heads;
wherein the flexible substrate is cut around the areas of the adhesive and the contact heads to form separate electrodes;
wherein the flexible substrate is cut around the contact pads to form a common lead connection hub; and
wherein the flexible substrate is cut between the conductive pathways to enable the conductive pathways to extend from the separate electrodes to the common lead connection hub.

12. The construct according to Claim 11, wherein at least five separate electrodes are formed on the flexible substrate.

13. The construct according to Claim 11, wherein the dielectric insulation layer is printed over the common deposit of conductive ink that forms the conductive pathways.

14. The construct according to Claim 11, wherein each of the conductive pathways has a different length between the separate electrodes and the common lead connection hub.
